# EUROPEAN PATENT APPLICATION

(11) **EP 1 403 302 A1**
(43) Date of publication of application: **31.03.2004**
(21) Application number: 02705290.1
(22) Date of filing: 18.03.2002
(51) Int. Cl.: C08G 63/78, C07C 67/00, C07C 69/68, C07D 323/00, C08G 63/08, C08L 67/04

(54) **PROCESS FOR PRODUCING LACTIC ACID OLIGOMER**

(30) Priority: 19.03.2001 JP 2001078215
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); MURAKAMI, Masahiro, Osaka-shi, Osaka 547-0026 (JP)
(74) Representative: Sternagel, Fleischer, Godemeyer & Partner Patentanwälte
(86) International application number: PCT/JP2002/002542
(87) International publication number: WO 2002/074835

(57) **Abstract**

An object of the present invention is to provide a novel method for the effective preparation of a mixture of linear and cyclic lactic acid oligomers. The present invention provides a method for the preparation of a mixture of linear and cyclic lactic acid oligomers represented by the following formula (1) or (2): 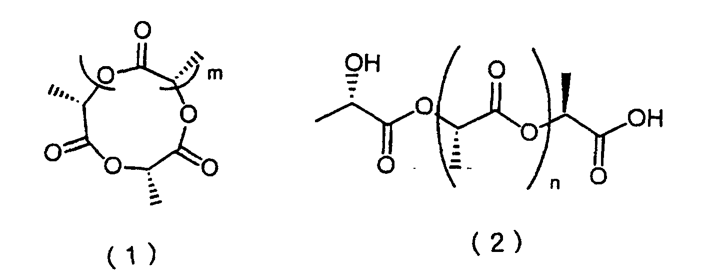 wherein m represents an integer of 1 to 30, and n represents an integer of 1 to 30, wherein lactides are polymerized in the presence of a compound represented by the following formula (3):

Me-N(R¹)(R²) (3)

wherein Me represents an alkali metal, and each of R¹ and R² independently represents an aliphatic group or aromatic group.

## Description

### TECHNICAL FIELD

The present invention relates to a method for the preparation of a mixture of linear and cyclic lactic acid oligomers, and a mixture of linear and cyclic lactic acid oligomers which is produced by the production method.

### BACKGROUND ART

A lactic acid oligomer is a useful compound, which is used as a medicament such as a tumor cell growth inhibiting agent (JP Patent Publication (Kokai) No. 3-193731 A (1991)) or antineoplastic agent (JP Patent Publication (Kokai) No. 9-227388 A (1997)), or an intermediate thereof.

The conventional method for producing such a lactic acid oligomer involves condensing lactic acids by heating dehydration under an inactive atmosphere, and then separating and collecting the oligomer component from the obtained reaction products.

However, since it is difficult to selectively produce a lactic acid oligomer by this conventional method and the lactic acid polymer obtained in the dehydration condensation process of lactic acids has a broad molecular weight distribution, containing high polymers, separation and collection of a lactic acid oligomer by separation means such as chromatography has been required.

### DISCLOSURE OF THE INVENTION

It is an object of the present invention is to provide a novel method for effective preparation of a mixture of linear and cyclic lactic acid oligomers. It is another object of the present invention is to provide a method for the preparation of a mixture of linear and cyclic lactic acid oligomers wherein the content of linear lactic acid oligomer is higher than that of cyclic lactic acid oligomer. It is another object of the present invention is to provide a mixture of linear and cyclic lactic acid oligomers which is produced by the above mentioned method.

As a result of intensive studies directed towards achieving the aforementioned objects, the present inventors have found that a mixture of linear and cyclic lactic acid oligomers can be effectively prepared by polymerizing lactides in the presence of a certain compound, thereby completing the present invention.

The present invention provides a method for the preparation of a mixture of linear and cyclic lactic acid oligomers represented by the following formula (1) or (2): 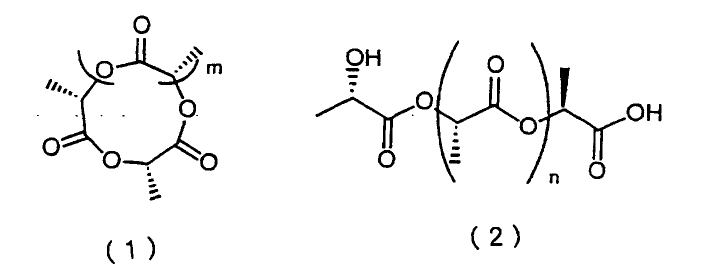 wherein m represents an integer of 1 to 30, and n represents an integer of 1 to 30,
wherein lactides are polymerized in the presence of a compound represented by the following formula (3):

Me-N(R¹)(R²) (3)

wherein Me represents an alkali metal, and each of R¹ and R² independently represents an aliphatic group or aromatic group.

Preferably, Me is lithium in the formula (3).

Preferably, each of R¹ and R² independently represents an alkyl group containing 1 to 6 carbon atoms in the formula (3).

Preferably, Me is lithium, and each of R¹ and R² is an isopropyl group in the formula (3).

Preferably, m is an integer of 1 to 19 in the formula (1).

Preferably, n is an integer of 1 to 25 in the formula (2).

According to another aspect of the present invention, there is provided a mixture of linear and cyclic lactic acid oligomers which is produced by the above production method of the present invention.

### BRIEF DESCRIPTION OF THE DRAWINGS

Figure 1 shows a general view of the positive mode FABMS spectrum of the product obtained in Example 1. Range: m/z 10.0000 to 1305.5900
Figure 2 shows a general view of the negative mode FABMS spectrum of the product obtained in Example 1. Range: m/z 10.0000 to 2000.0000
Figure 3 shows an enlarged view of the negative mode FABMS spectrum of the product obtained in Example 1. Range: m/z 10.0000 to 501.9260
Figure 4 shows an enlarged view of the negative mode FABMS spectrum of the product obtained in Example 1. Range: m/z 490.2980 to 1003.7700
Figure 5 shows an enlarged view of the negative mode FABMS spectrum of the product obtained in Example 1. Range: m/z 999.9500 to 1504.3400
Figure 6 shows an enlarged view of the negative mode FABMS spectrum of the product obtained in Example 1. Range: m/z 1484.5300 to 2000.0000
Figure 7 shows a general view of the NMR spectrum of the product obtained in Example 1.

### THE BEST MODE FOR CARRYING OUT THE INVENTION

The embodiments and methods for carrying out the present invention are described in detail below.

The method for the preparation of a mixture of linear and cyclic lactic acid oligomers according to the present invention is characterized in that lactides are polymerized in the presence of a compound represented by the following formula (3):

Me-N(R¹)(R²) (3)

wherein Me represents an alkali metal, and each of R¹ and R² independently represents an aliphatic group or aromatic group.

The starting material used in the production method of the present invention is lactide (3,6-dimethyl-1,4-dioxane-2,5-dione) obtained by condensation of two molecules of lactic acid by dehydration, and the lactides are allowed to react in the presence of an alkali metal compound represented by the above formula (3). The formula (3):

Me-N(R¹)(R²) (3)

is explained below.

In the formula (3), Me represents an alkali metal, and each of R¹ and R² independently represents an aliphatic group or aromatic group.

Examples of the aliphatic group defined in the present specification include a straight chain, branched chain, cyclic, or their combined form, saturated or unsaturated aliphatic hydrocarbon group containing 1 to 12, and preferably 1 to 6 carbon atoms. Specific examples include alkyl groups such as methyl, ethyl, n-propyl, i-propyl, n-butyl, i-butyl, t-butyl, octyl and dodecyl, and cycloalkyl groups such as cyclopropyl, cyclobutyl, cyclooctyl and cyclododecyl. The aliphatic group may be an unsaturated hydrocarbon group having a double or triple bond.

Examples of the aromatic group defined in the present invention include an aryl group and an arylalkyl group, containing 6 to 30, preferably 6 to 20, more preferably 6 to 12, and further more preferably 6 to 10 carbon atoms. Examples of the aryl group include phenyl, tolyl and naphthyl, and examples of the arylalkyl group include benzyl, phenethyl and naphthylmethyl.

The aliphatic group and the aromatic group may have one or more substituent(s). The type of substituents is not particularly limited, and the examples include a straight chain, branched chain, linear or cyclic alkyl group, a straight chain, branched chain, linear or cyclic alkenyl group, a straight chain, branched chain, linear or cyclic alkynyl group, an aryl group, an acyloxy group, an alkoxycarbonyloxy group, an aryloxycarbonyloxy group, a carbamoyloxy group, a carbonamide group, a sulfonamide group, a carbamoyl group, a sulfamoyl group, an alkoxy group, an aryloxy group, an aryloxycarbonyl group, an alkoxycarbonyl group, an N-acylsulfamoyl group, an N-sulfamoylcarbamoyl group, an alkylsulfonyl group, an arylsulfonyl group, an alkoxycarbonylamino group, an aryloxycarbonylamino group, an amino group, an ammonio group, a cyano group, a nitro group, a carboxyl group, a hydroxyl group, a sulfo group, a mercapto group, an alkylsulfinyl group, an arylsulfinyl group, an alkylthio group, an arylthio group, an ureide group, a heterocyclic group (e.g., a monocyclic or condensed ring containing at least one or more nitrogen, oxygen or sulfur atom(s) and consisting of 3 to 12 ring forming members), a heterocyclic oxy group, a heterocyclic thio group, an acyl group, a sulfamoylamino group, a silyl group, and a halogen atom. In the above, the carbon number of alkyl, alkenyl, alkynyl and alkoxy is generally 1 to 12, and preferably 1 to 6, and the carbon number of aryl is generally 6 to 20, and preferably 6 to 10.

In the formula (3), Me represents an alkali metal. Examples of an alkali metal include Li, Na and K, and Li is preferred.

Among the compounds represented by the formula (3), the compounds having asymmetric carbon atoms may be any one of (R) form, (S) form, and (R),(S) form.

A method for obtaining an alkali metal compound represented by the formula (3) is not particularly limited, and a person skilled in the art can obtain the compound as appropriate. For example, the alkali metal compound can be obtained by reaction of dialkylamine such as diisopropylamine with an alkylated alkali metal such as n-butyllithium. More specifically, this reaction can be carried out, for example, by mixing a solution containing dialkylamine in an inert solvent such as THF with a solution containing an alkylated alkali metal in an inert solvent such as hexane under conditions that are inactive for the reaction, e.g., under a nitrogen gas atmosphere, and then stirring the mixture. The reaction temperature is not particularly limited, as long as the reaction progresses, but it is preferably -78°C to room temperature. The reaction temperature can be set as appropriate.

When lactides are polymerized in the presence of a compound represented by the formula (3) according to the method of the present invention, the used amount of the compound represented by the formula (3) (Me-N(R¹)(R²)) is preferably 0.1 to 1 mol, and more preferably 0.2 to 0.3 mol per mole of lactide.

When the method of the present invention is carried out, the reaction temperature is not particularly limited as long as the reaction progresses, but it is preferably -100°C to room temperature, and more preferably -78°C to room temperature.

Polymerization reaction of lactides in the method of the present invention is preferably carried out in the presence of a reaction solvent. The reaction solvent is not particularly limited as long as it is inactive for the reaction. Examples of preferred solvents include cyclic ethers such as tetrahydrofuran, diethylether, and dimethoxyethane. Examples of reaction atmospheres to be used may include inactive gas atmospheres such as nitrogen gas and argon gas. Reaction pressure is not particularly limited, and it is preferably normal pressure.

The composition of the mixture of linear and cyclic lactic acid oligomers which is obtained by the method of the present invention is altered depending on the type of the compound of the formula (3) used as a reaction assistant and the reaction conditions. Preferably, the content of linear lactic acid oligomer is higher than that of cyclic lactic acid oligomer.

According to the method of the present invention, there is produced a mixture of linear and cyclic lactic acid oligomers represented by the following formula (1) or (2): 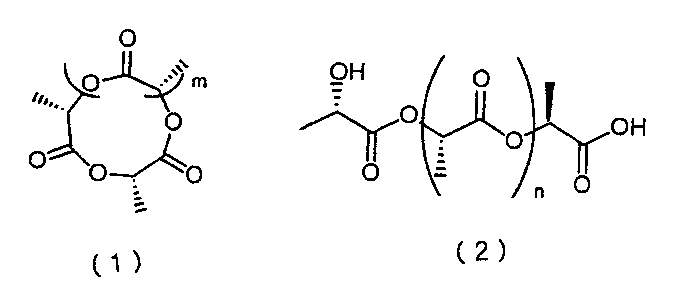 wherein m represents an integer of 1 to 30, and n represents an integer of 1 to 30.

The reaction product obtained by the method of the present invention is generally a mixture of a cyclic lactic acid oligomer wherein m represents an integer ranging from 1 to 30, e.g., 1 to 28, 1 to 25, 1 to 21, 1 to 19, etc., and a linear lactic acid oligomer wherein n represents an integer ranging from 1 to 30, e.g., 1 to 28, 1 to 25, etc. According to the present invention, there is provided a mixture of linear and cyclic lactic acid oligomers which is produced by the above-described production method according to the present invention.

The mixture of linear and cyclic lactic acid oligomers produced by the method of the present invention (or a lactic acid oligomer consisting of a single substance obtained by purification from the mixture) is useful as a tumor cell growth inhibiting agent, an antineoplastic agent, a preventive agent against cancer metastasis, a QOL improving agent for cancer patients, an immunoreactive agent, and the like. The mixture can also be used for prevention and/or treatment of diabetes or diabetes complications, since it has an action of reducing blood sugar level. Moreover, the mixture of cyclic lactic acid oligomers produced by the method of the present invention (or a single substance obtained by purification from the mixture) acts to repress excessive appetite and to promote basal metabolism, and so it can be used also as a medicament useful for improvement and/or prevention of adiposis and enhancement of effects of kinesitherapy. It is also useful as an agent for promoting glycogen accumulation or an agent for enhancing physical fitness.

Furthermore, the mixture of linear and cyclic lactic acid oligomers produced by the method of the present invention (or a lactic acid oligomer consisting of a single substance obtained by purification from the mixture) is useful not only as a medicament, but also as health foods or diet supplements including beverages, etc., which are generally called soft drinks, drinkable preparations, health foods, specific hygienic foods, functional foods, foods for function activation, nutritional supplementary foods, supplements, feed, feed additives, etc.

All of the contents disclosed in the specification of Japanese Patent Application No. 2001-78215, based on which the present application claims a priority, are incorporated herein as a part of the disclosure of this specification.

The present invention will be described in detail by the following examples, but the present invention is not limited thereto.

The present invention is further described in the following example. It is apparent to those skilled in the art that materials, usage, proportion, treatment, treatment process, etc. shown in the following example can be modified as appropriate, as long as the modifications are within the spirit and scope of the invention, and the scope of the present invention is not limited to the following examples.

### EXAMPLE

### Example 1.

The reaction scheme of Example 1 is shown below.

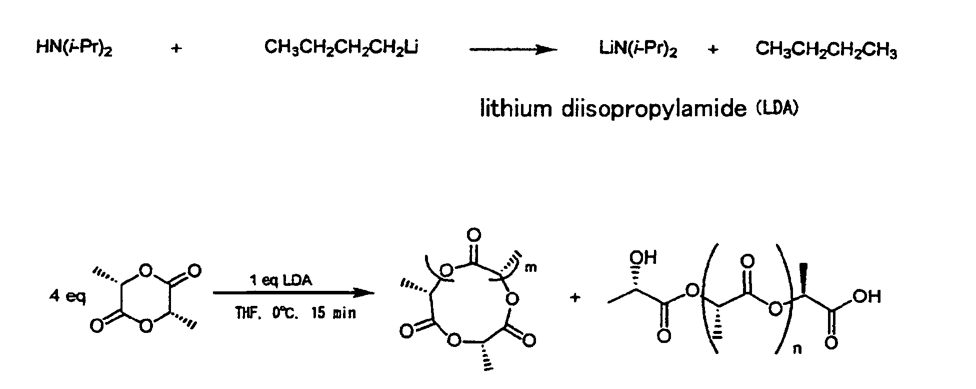

0.63 ml of n-butyllithium (1.6 M hexane solution, 1 mmol) was added to a 5 ml THF solution containing 0.101 g (1 mmol) of diisopropylamine at 0°C under a nitrogen gas atmosphere, and the obtained mixture was stirred for 10 minutes, so as to obtain lithium diisopropylamide (LDA). Thereafter, 4 ml of THF solution containing 0.577 g (4 mmol) of L-(-)-lactide was added thereto followed by stirring for 15 minutes for reaction. Thereafter, 20 ml of a saturated ammonium chloride aqueous solution was added to the obtained reaction mixture to treat the reaction, and 10 ml of water was further added thereto. Extractions were carried out 5 times with THF (50 ml), and the organic layer was dried with anhydrous sodium sulfate. After anhydrous sodium sulfate was filtrated, the organic solvent was subjected to vacuum concentration, so as to obtain 0.53 g of a crude product. 6 ml of ether was added to the obtained crude product, and the mixture was immersed in an ultrasonic cleaner for 10 minutes for filtration, so as to obtain 0.39 g of a white solid product having a melting point of 125°C to 129°C.

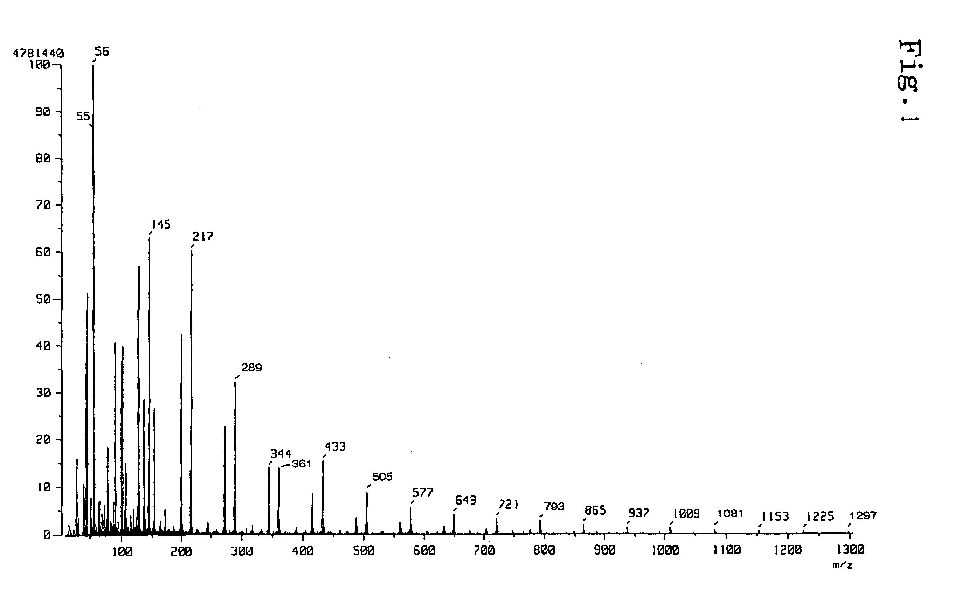

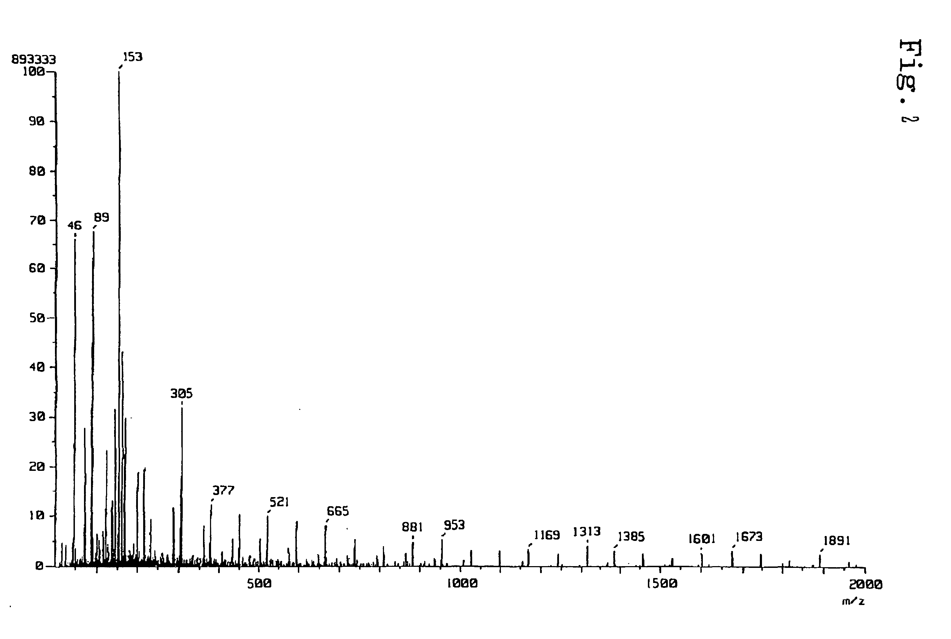

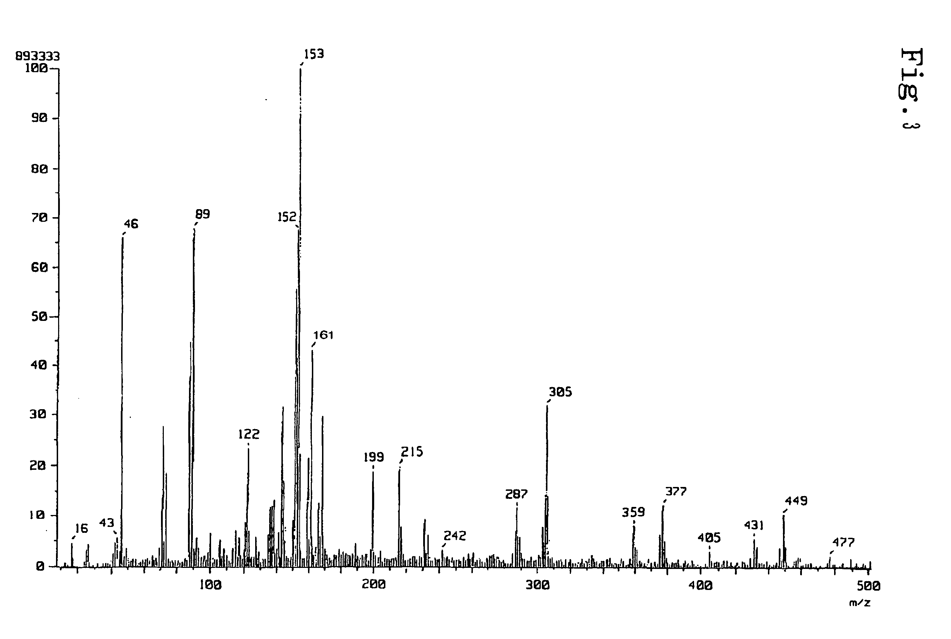

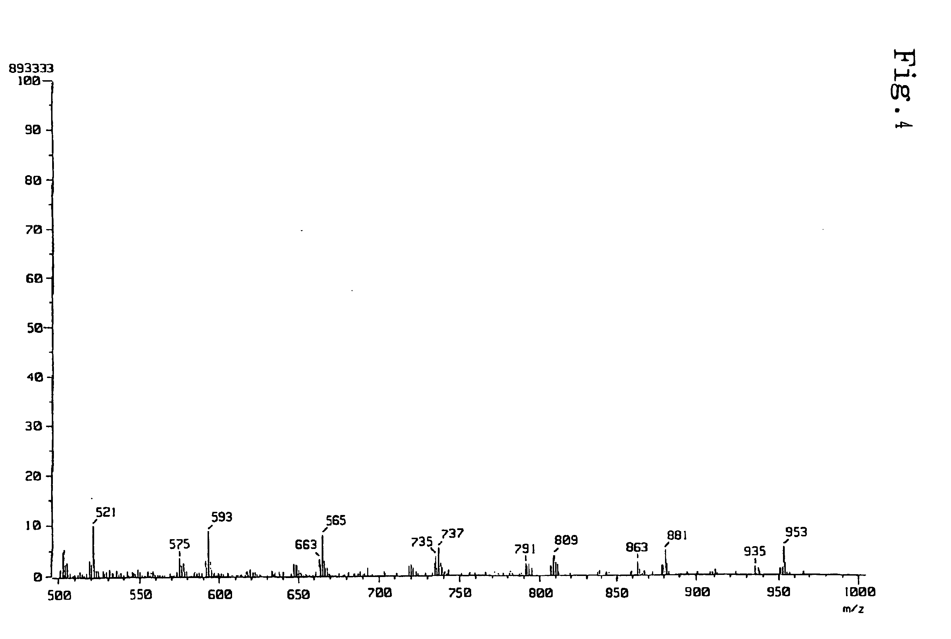

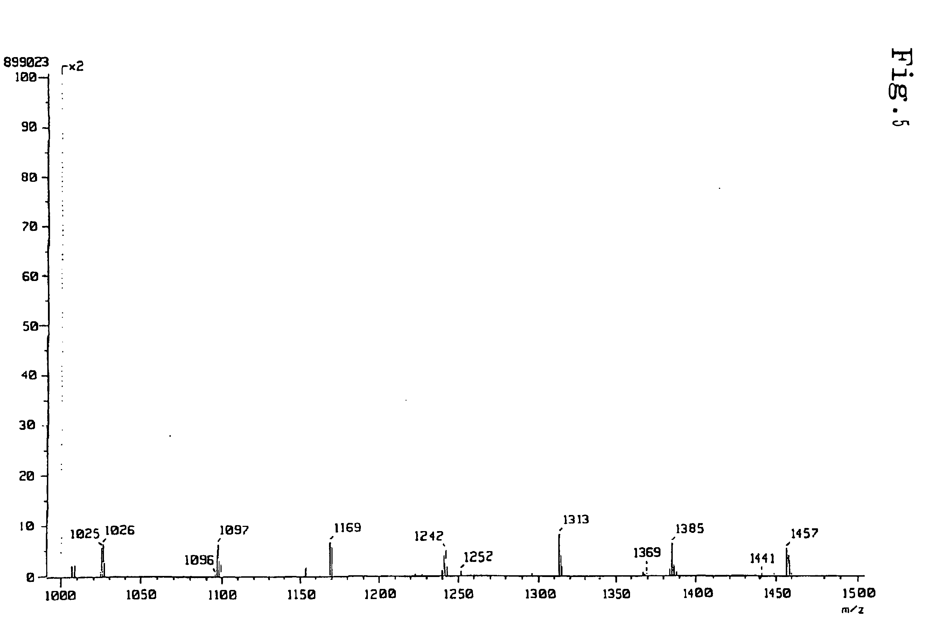

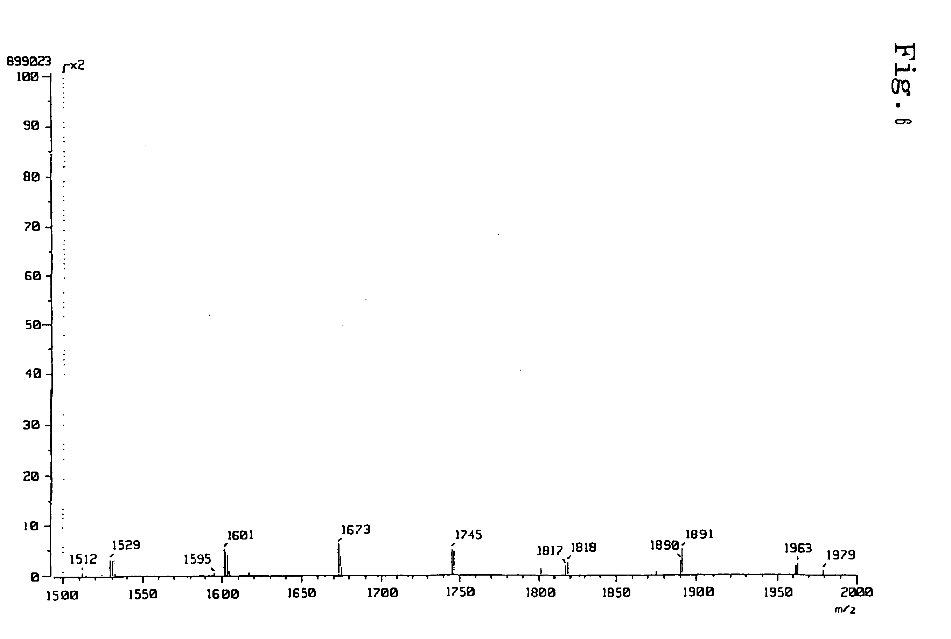

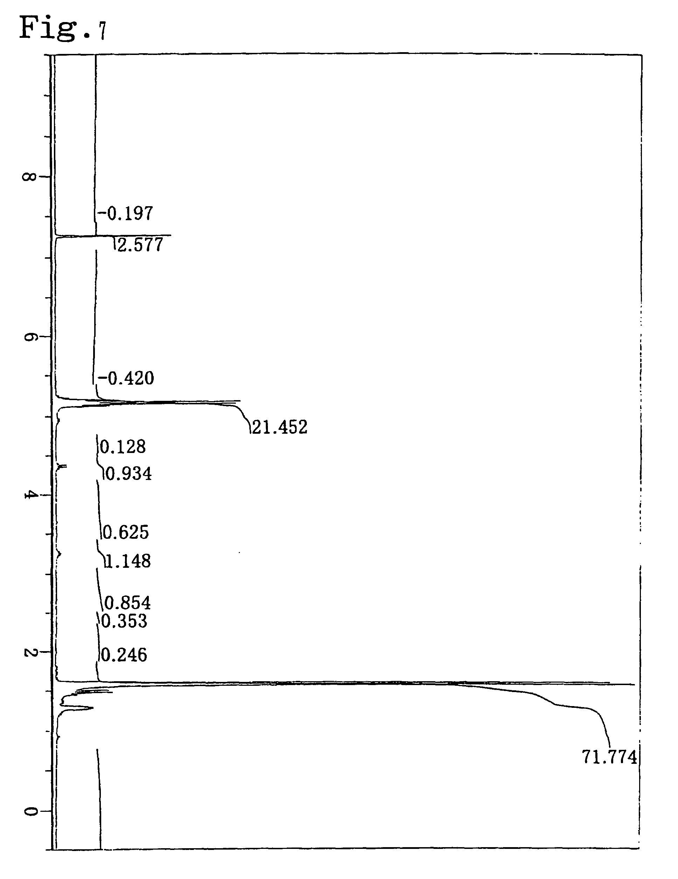

The physical data of the obtained product are shown in Figures 1 to 7. From the FABMS and NMR data shown in Figures 1 to 7, it was confirmed that a 3-mer to 21-mer cyclic lactic acid oligomer and a 3-mer to 27-mer linear lactic acid oligomer were present in the solid product.

### INDUSTRIAL APPLICABILITY

According to the method for the preparation of a mixture of linear and cyclic lactic acid oligomers according to the present invention, a lactic acid oligomer can be produced with good yield, which has great industrial significance. In addition, the mixture of linear and cyclic lactic acid oligomers which is produced by the production method of the present invention is useful as a tumor cell growth inhibiting agent, antineoplastic agent, preventive agent against cancer metastasis, QOL improving agent for cancer patients, immunoreactive agent, therapeutic agent for diabetes, antiobestic agent, an agent for promoting glycogen accumulation, or an agent for enhancing physical fitness. Furthermore, the mixture of linear and cyclic lactic acid oligomers is useful not only as a medicament, but also for various types of health foods and diet supplements including soft drinks, drinkable preparations, health foods, specific hygienic foods, functional foods, foods for function activation, nutritional supplementary foods, supplements, feed, feed additives, etc.

## Claims

1. A method for the preparation of a mixture of linear and cyclic lactic acid oligomers represented by the following formula (1) or (2): 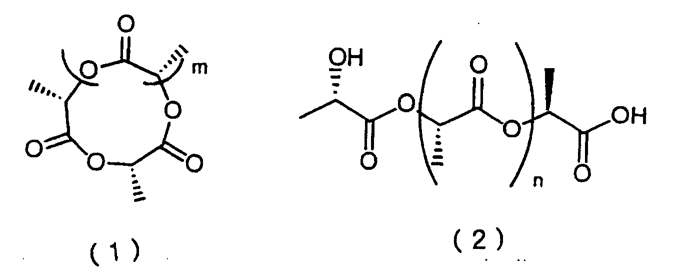 wherein m represents an integer of 1 to 30, and n represents an integer of 1 to 30, wherein lactides are polymerized in the presence of a compound represented by the following formula (3):
Me-N(R¹)(R²) (3)
wherein Me represents an alkali metal, and each of R¹ and R² independently represents an aliphatic group or aromatic group.

2. The method according to claim 1 wherein Me is lithium in the formula (3).

3. The method according to claim 1 or 2 wherein each of R¹ and R² independently represents an alkyl group containing 1 to 6 carbon atoms in the formula (3).

4. The method according to any one of claims 1 to 3 wherein Me is lithium, and each of R¹ and R² is an isopropyl group in the formula (3).

5. The method according to any one of claims 1 to 4 wherein m is an integer of 1 to 19 in the formula (1).

6. The method according to any one of claims 1 to 5 wherein n is an integer of 1 to 25 in the formula (2).

7. A mixture of linear and cyclic lactic acid oligomers which is produced by the production method according to any one of claims 1 to 6.
